# FASCICULE DE BREVET EUROPEEN

(11) **EP 2 498 786 B1**
(45) Date de publication et mention de la délivrance du brevet: **27.01.2016**
(21) Numéro de dépôt: 10795413.3
(22) Date de dépôt: 03.11.2010
(51) Int. Cl.: A61K 33/00, A61P 25/14

(54) **MÉDICAMENT INHALABLE À BASE DE XÉNON POUR TRAITER OU POUR PRÉVENIR LES DYSKINÉSIES INDUITES**
AUF XENON BASIERENDES INHALIERBARES ARZNEIMITTEL ZUR BEHANDLUNG ODER PRÄVENTION VON INDUZIERTER DYSKINESIE
XENON-BASED INHALABLE DRUG FOR TREATING OR PREVENTING INDUCED DYSKINESIA

(30) Priorité: 10.11.2009 FR 0957921
(43) Date de publication de la demande: 19.09.2012
(73) Titulaire: L'Air Liquide Société Anonyme pour l'Etude et l'Exploitation des Procédés Georges Claude, 75007 Paris (FR)
(72) Inventeur: BESSIERE, Baptiste, F-92130 Issy Les Moulineaux (FR)
(74) Mandataire: Pittis, Olivier
(86) Numéro de dépôt international: PCT/FR2010/052350
(87) Numéro de publication internationale: WO 2011/058262

(56) Documents cités:
- WO-A2-2005/011711
- US-B1- 6 274 633
- US-B1- 6 559 190
- JENNER PETER: "Molecular mechanisms of L-DOPA-induced dyskinesia", NATURE REVIEWS NEUROSCIENCE, vol. 9, no. 9, septembre 2008 (2008-09), pages 665-677, XP008121207, ISSN: 1471-0048 cité dans la demande
- JANSEN M ET AL: "Antagonists and agonists at the glycine site of the NMDA receptor for therapeutic interventions", EUROPEAN JOURNAL OF MEDICINAL CHEMISTRY, EDITIONS SCIENTIFIQUE ELSEVIER, PARIS, FR LNKD- DOI:10.1016/S0223-5234(03)00113-2, vol. 38, no. 7-8, 1 juillet 2003 (2003-07-01), pages 661-670, XP004448317, ISSN: 0223-5234
- DINSE A ET AL: "Xenon reduces glutamate-, AMPA-, and kainate-induced membrane currents in cortical neurones", BRITISH JOURNAL OF ANAESTHESIA, vol. 94, no. 4, avril 2005 (2005-04), pages 479-485, XP008121311, ISSN: 0007-0912
- PARSONS C G ET AL: "GLUTAMATE IN CNS DISORDERS AS A TARGET FOR DRUG DEVELOPMENT: AN UPDATE", DRUG NEWS AND PERSPECTIVES, PROUS SCIENCE LNKD- DOI:10.1358/DNP.1998.11.9.863689, vol. 11, no. 9, 1 janvier 1998 (1998-01-01), pages 523-569, XP000910825, ISSN: 0214-0934

## Description

L'invention porte sur un médicament gazeux à base de xénon gazeux permettant, après inhalation de prévenir ou de traiter une dyskinésie chez le mammifère, en particulier chez l'être humain, induite par lévodopa utilisée dans le traitement de la maladie de Parkinson.

Les dyskinésies sont des troubles se traduisant par des mouvements involontaires anormaux. Il en existe plusieurs types, notamment :
- les chorées : mouvements irréguliers, rapides et brusques qui affectent le visage, les bras, les jambes ou le tronc, par exemple la chorée de Huntington,
- les ballismes : mouvements analogues à ceux des chorées mais plus violents et de plus grande force,
- les dystonies : contractions musculaires soutenues produisant habituellement des torsions et des mouvements répétitifs et des positions ou postures anormales, et
- les athétoses : mouvements involontaires lents, sinueux et non coordonnées des membres surtout des mains, du tronc ou du visage.

Les mouvements et autres troubles sont dus à des dysfonctionnements des ganglions de la base et des structures du cerveau associées. De tels troubles peuvent survenir suite à des maladies héréditaires ou acquises, des neuro-dégénérescences idiopathiques ou peuvent être iatrogéniques.

Le spectre de troubles est donc très large et comprend non seulement ceux liés à la puissance des mouvements (akinésie, hypokinésie, bradykinésie) et les hypertonies (e.g. maladie de Parkinson, certaines formes de dystonies...) mais aussi les troubles de mouvements involontaires (hyperkinésies, dyskinésies,...).

La connaissance des mécanismes pathophysiologiques liés à ces troubles rend probable le fait que des mécanismes similaires soient médiateurs des troubles caractérisés par soit les hyperkinésies, soit des dyskinésies. En d'autres termes, les traitements efficaces contre une forme de dyskinésie sont susceptibles d'être aussi efficaces contre d'autres formes de dyskinésies ayant une autre étiologie. Toutefois, cela reste encore à être démontré.

En fait, comme le rappelle le document de O. Rascol et coll.; Dyskinesia: L-dopa-induced and tardive dyskinesia ; Clin. Neuropharmacol.; 2001; Nov-Dec;24(6):313-23; il existe deux types principaux de dyskinésies, à savoir les dyskinésies tardives induites par des neuroleptiques et les dyskinésies induites par la L-dopa ou lévodopa, lesquelles conduisent toutes les deux à des mouvements involontaires et anormaux des personnes qui en sont atteintes.

Cependant, les similitudes s'arrêtent là puisque ce document précise clairement que ces deux types de dyskinésies sont totalement différents en ce qui concerne non seulement les composés qui les induisent mais aussi les maladies sous-jacentes.

Concernant les dyskinésies induites par la L-dopa ou d'autres agonistes dopaminergiques analogues, l'apparition de ces dyskinésies se produit en tant qu'effet secondaire des thérapies de remplacement de la dopamine mises en oeuvre contre les troubles des mouvements liés aux ganglions de la base ou le syndrome de Parkinson, comme décrit par le document : Molecular mechanisms of L-DOPA-induced dyskinesia, Peter J., Nature Reviews Neuroscience, 2008.

En effet, les symptômes du syndrome de Parkinson se caractérisent par des mouvements lents (i.e. des bradykinésies), des rigidités et des tremblements.

Dans la maladie de Parkinson, la pathologie primaire est une dégénérescence des neurones dopaminergiques de la *substantia nigra, pars compacta.*

Le traitement symptomatique le plus répandu actuellement du syndrome de Parkinson est basé sur l'usage d'agents de remplacements de la dopamine, e.g. des agonistes des récepteurs à dopamine, en particulier la lévodopa, habituellement dénommée L-DOPA.

Ceux-ci ont cependant des inconvénients, en particulier lors des traitements à long terme, car leur usage peut engendrer, notamment une érosion de l'effet anti-parkinsonien du traitement et l'apparition d'effets secondaires qui se traduisent par des dyskinésies induites, telles que chorées et dystonies.

Dans ce cas, les dyskinésies sont donc des effets secondaires des traitements de la maladie de Parkinson au moyen de L-dopa ou d'autres agonistes dopaminergiques analogues.

Or, ces effets secondaires limitent l'usage et l'intérêt de ces traitements dopaminergiques.

Par ailleurs, l'autre cause de dyskinésies est le traitement des psychoses avec des drogues neuroleptiques qui engendrent des dyskinésies induites par des neuroleptiques, connues sous le nom de dyskinésies tardives.

A ce jour, de nombreux traitements des dyskinésies ont été proposés mais ceux-ci ne se sont pas révélés vraiment efficaces ou alors engendraient des effets secondaires indésirables.

Ainsi, les documents WO-A-2005/011711 et US-A-6559190 enseignent l'utilisation possible de xénon inhalé pour traiter ou prévenir les dyskinésies tardives. Toutefois, ces documents n'abordent pas la question du traitement des dyskinésies induites par l'utilisation d'un agoniste dopaminergique, telle la lévodopa.

Le problème qui se pose dès lors est de proposer un médicament et un traitement efficaces des dyskinésies se produisant chez le mammifère, en particulier chez l'être humain, lesquelles résultent ou sont induites par l'utilisation de la lévodopa, qui est utilisée dans le traitement de la maladie de Parkinson.

En d'autres termes, la présente invention ne vise pas à soigner la maladie de Parkinson en tant que telle mais à pallier aux effets négatifs engendrés par la L-Dopa, utilisée dans le traitement de la maladie de Parkinson, laquelle est susceptible d'engendrer ou d'induire des dyskinésies particulières en réaction à son utilisation dans le traitement de la maladie de Parkinson.

La solution de l'invention est alors basée sur un médicament gazeux à base de xénon gazeux pour une utilisation par inhalation pour traiter ou prévenir une dyskinésie chez le mammifère, ladite dyskinésie étant induite par la L-Dopa.

Selon le cas, le médicament gazeux inhalable selon l'invention peuvent comprendre l'une ou plusieurs des caractéristiques suivantes :
- la dyskinésie est induite par la L-Dopa
- le mammifère est un être humain, c'est-à-dire un homme ou une femme, y compris les adolescents ou tout autre groupe d'individus.
- le xénon gazeux est mélangé avec un gaz contenant de l'oxygène, en particulier le xénon est mélangé avec de l'air ou un mélange N₂/O₂.
- il contient une proportion volumique de xénon d'au moins 5% en volume et/ou inférieure à 50% en volume, c'est-à-dire typiquement comprise entre 5 et 50%, de préférence inférieure ou égale à 40%.
- il contient une proportion volumique de xénon d'au moins 10% en volume et/ou inférieure ou égale à 30% en volume de préférence entre 10 et 30%.
- le xénon est mélangé avec au moins 21% en volume d'oxygène:
- le xénon est mélangé avec au moins un autre composé choisi parmi le protoxyde d'azote (N₂O), l'argon, hélium, néon, krypton, H₂S, CO, NO et l'azote.
- le xénon est administré en combinaison avec la lévodopa.
- le xénon est administré préalablement, simultanément et/ou postérieurement à l'administration de l'agent de remplacement de la dopamine. Par exemple, le xénon peut être administré simultanément à l'agent de remplacement de la dopamine, notamment sous forme d'un aérosol ou d'une suspension comprenant du xénon gazeux et l'agent de remplacement de la dopamine sous forme liquide ou solide.
- le médicament inhalable est administré par inhalation pendant une durée d'administration allant de quelques minutes à quelques heures et/ou à une fréquence pouvant atteindre une à plusieurs fois par jour ou par semaine.

Plus généralement, le xénon gazeux selon l'invention sert donc à fabriquer un médicament inhalable destiné à traiter ou à prévenir une dyskinésie induite, lequel médicament peut comprendre tout ou partie des caractéristiques susmentionnées.

Dit autrement, selon l'invention, on administre par inhalation du xénon gazeux à un individu, c'est-à-dire un homme ou une femme, de manière à traiter ou à prévenir une dyskinésie chez cet individu, en particulier une dyskinésie résultant ou induite par la lévodopa. De préférence, le médicament inhalable est sous forme gazeuse et contient de 5 à 40% en volume de xénon et de l'oxygène.

La présente invention repose donc sur un traitement des dyskinésies basé sur une action sur les récepteurs N-methyl-D-aspartate (NMDA). En fait, les récepteurs NMDA sont une classe de récepteurs à acides aminés excitateurs ayant de nombreuses fonctions importantes dans le circuit moteur des ganglions de la base. Ainsi, le document : Rationale for and use of NMDA receptor antagonists in Parkinson's disease, Hallett P. et al. Pharmacology & Therapeutics, 102 (155-174), 2004*,* envisage l'utilisation de ces récepteurs dans le traitement de la maladie de Parkinson.

En effet, le rôle possible des récepteurs NMDA dans les dyskinésies est supporté par l'action des antagonistes des récepteurs NMDA, par exemple dextrophan et dextromethorman, chez l'homme et une gamme d'antagonistes des récepteurs NMDA chez les primates traités par MPTP (1-méthyl-4-phényl-1,2,3,6-tétrahydropyridine), qui est un puissant neurotoxique sélectif des neurones dopaminergiques (modèle animaux de la maladie de Parkinson), comme le montre le document Molecular mechanisms of L-DOPA-induced dyskinesia, Peter J., Nature Reviews Neuroscience, 2008), puisque ces substances réduisent significativement les mouvements involontaires (dyskinésies) mais en engendrant par contre des effets secondaires importants.

Afin de montrer l'efficacité du xénon dans le traitement, c'est-à-dire la réduction, des dyskinésies induite par la lévodopa, on a utilisé le protocole expérimental suivant qui est basé sur une induction d'une dyskinésie par de la lévodopa dans un modèle animal de la maladie de Parkinson. Ce modèle, bien admis dans la littérature, est classiquement utilisé afin d'identifier les futures traitements contre les dyskinésies.

Les résultats d'essais sont illustrés sur les figures ci-jointes parmi lesquelles :
- la Figure 1 schématise les sites d'injection dans les cerveaux de rats.
- la Figure 2 montre un rotomètre automatique utilisé pour vérifier que les animaux sont bien parkinsoniens.
- les Figures 3A et 3B illustrent les effets du xénon sur les dyskinésies axiales chez le rat parkinsonien.
- les Figures 4A et 4B illustrent les effets du xénon sur les dyskinésies des membres inférieurs chez le rat parkinsonien.
- les Figures 5A et 5B illustrent les effets du xénon sur les dyskinésies orolinguales chez le rat parkinsonien.
- et les Figures 6A et 6B illustrent les effets du xénon sur les dyskinésies locomotrices chez le rat parkinsonien.

Plus précisément, les effets de l'administration de plusieurs gaz en tant que traitement des dyskinésies ont été évalués chez des rats ayant des lésions engendrées par l'injection unilatérale de 6-OHDA (6-hydroxy-dopamine, puissant neurotoxique) au niveau d'une voie ascendante nigro-striatale (modèle animal de la maladie de Parkinson).

Dans ce modèle, le traitement chronique des rats avec de la lévodopa induit des mouvements anormaux involontaires (MAIs) qui sont analogues à ceux des dyskinésies induites par lévodopa chez l'homme.

Le but des essais est donc de démontrer que le xénon inhalé permet de supprimer ou au moins de limiter l'apparition de ces MAIs résultant ou induits par la lévodopa injectée aux rats, c'est-à-dire induits par la molécule servant au traitement anti-parkinsonien de ces rats.

### Protocole expérimental et résultats

Les rats à lésion induite par le 6-OHDA servant de modèle de la maladie de Parkinson sont préparés de la manière suivante.

16 rats Sprague-Dawley pesant entre 250 et 300 g sont conservés dans une pièce à température contrôlée (environ 22 °C) selon des cycles jour/nuit de 12 h, avec accès libre à la nourriture et à l'eau. Les animaux subissent, 30 min avant l'intervention, une injection intrapéritonéale de pargyline (5 mg/kg; inhibiteur de la monoamine oxydase-B) et de desipramine (25 mg/kg; inhibiteur de la recapture de la noradrénaline).

Les animaux sont placés dans une chambre d'anesthésie avec flux continu d'oxygène à 1.5 l/minute et 3% d'isoflurane. Après l'anesthésie, les animaux sont placés dans un cadre stéréotaxique.

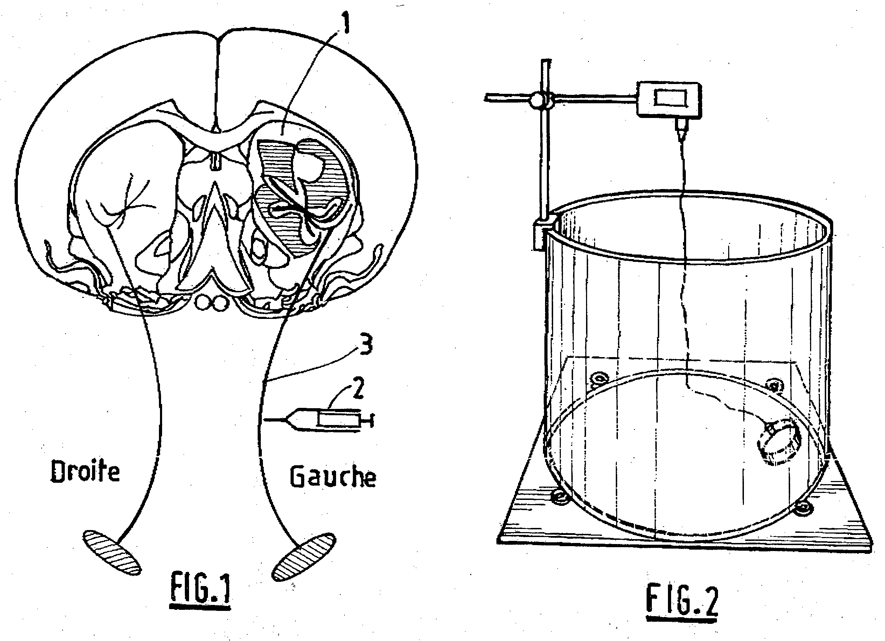

Comme visible sur la Figure 1, on injecte manuellement (voie ascendante nigrostriatale 3 sur Fig. 1) de la 6-Hydroxydopamine (6-OHDA) avec 0.1% d'acide ascorbique (5mg/ml) dissout dans de l'eau stérile en utilisant une seringue Hamilton 2 de 5 µl dans le faisceau médian du cerveau antérieur droit (en 1) à 5 µl/minute, sur une durée de 5 minutes. Le site d'injection est localisé selon les coordonnées relatives de bregma : -2.8 mm rostral, 2 mm latéral et 9 mm sous le crâne (Paxinos and Watson, 1986). On laisse les rats récupérer pendant 3 semaines après les lésions.

Les animaux reçoivent 0.05mg/kg s.c. d'apomorphine. Les animaux n'effectuant pas 50 rotations complètes sur le rotomètre automatique (voir Figure 2) dans l'heure qui suit sont retirés de l'étude.

Les animaux restant reçoivent une administration de 6 mg/kg de lévodopa et de 15mg/kg de benzerazide (i.p.), deux fois par jour, pendant 21 jours consécutifs. Pendant ces 21 jours, on évalue les mouvements involontaires anormaux (MIA) aux jours 1, 7, 14 et 21.

Les traitements thérapeutiques commencent au 22^{e} jour. Ils consistent en l'administration aux rats par inhalation de :
- mélanges gazeux xénon/O₂ (50%/50% en volume) en tant que gaz test selon l'invention et ;
- mélanges gazeux N₂/O₂ (50%/50% en volume) en tant que témoin (contrôle).

Les mélanges gazeux sont des pré-mélanges fournis par Air Liquide™. Les concentrations en gaz sont monitorées en permanence.

L'exposition aux gaz se fait dans une chambre en plexiglas de 42 cm de long, 26 cm de largeur et de hauteur. A chaque fois, 4 ou 5 rats sont mis dans la chambre et inhalent le gaz testé pendant 1h. La chambre est alimentée en gaz frais à un débit de 41/min.

On évalue les mouvements involontaires anormaux (MIA) pendant 180 min après l'injection de levodopa. Plus précisément, les animaux sont placés dans des boites (22 cm x 34 cm x 20 cm) et y sont laissés pendant 15 minutes pour qu'ils s'habituent à leur environnement. Du lévodopa méthyl ester (6mg/kg, i.p.) est alors administré à des intervalles de temps de 1 minute entre les rats. Chaque rat est observe pendant 1 minute à des intervalles de 30 minutes après injection et pendant une période de 180 min.

Quatre sous-types de MAIs sont évalués, à savoir :
- Locomoteur (Lo) : mouvements accrus controlatérales à la lésion,
- Membres inférieurs (Li) : mouvements incontrôlés aléatoires des membres inférieurs controlatérales à la lésion,
- Orolinguales (Ol) : mastication et mouvements de machoire excessifs avec sortie de langue,
- Axial (Ax) : postures dystoniques ou torsion chroéiforme du cou et du haut du corps du côté controlatérales.

La gravité des MIAs est notée (score) de 1 à 4 selon la durée des MIAs sur une période d'observation d'une minute :
- 1 = présence de MIAs pendant moins de 30 sec
- 2 = présence de MIAs pendant plus de 30 sec
- 3 = présence de MIAs pendant la minute avec interruption par stimuli externes
- 4 = présence de MIAs pendant la minute sans interruption par stimuli externes

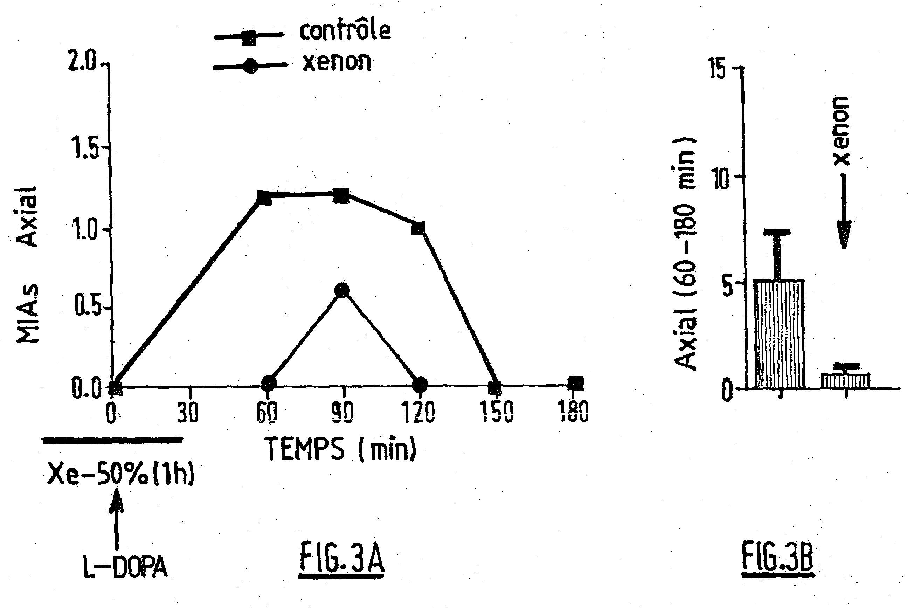

Les résultats obtenus confirment que les animaux parkinsoniens recevant de la L-Dopa développent les 4 types de dyskinésies : Lo, Li, Ol, Ax. Ceci est visible sur les courbes des groupes « contrôle » respirant le mélange gazeux sans xénon (mélange N₂/O₂ 50%/50%) illustrées sur les figures 3 à 6.

En effet, les Figures 3A et 3B illustrent les effets du xénon sur les dyskinésies axiales chez le rat parkinsonien. Comme on voit en Figure 3A, la cinétique de l'effet du xénon sur les dyskinésies axiales suite à l'administration de L-Dopa (à t=0) montre une diminution net de ces dyskinésies, tant en amplitude qu'en durée, par rapport au groupe contrôle ayant respiré le mélange gazeux ne contenant pas de xénon.

Par ailleurs, en Figure 3B, la moyenne des dyskinésies axiales de 60 à 180 min fait apparaître que, globalement, les rats ayant respirés le mélange contenant du xénon présente moins de dyskinésie axiales (Axial).

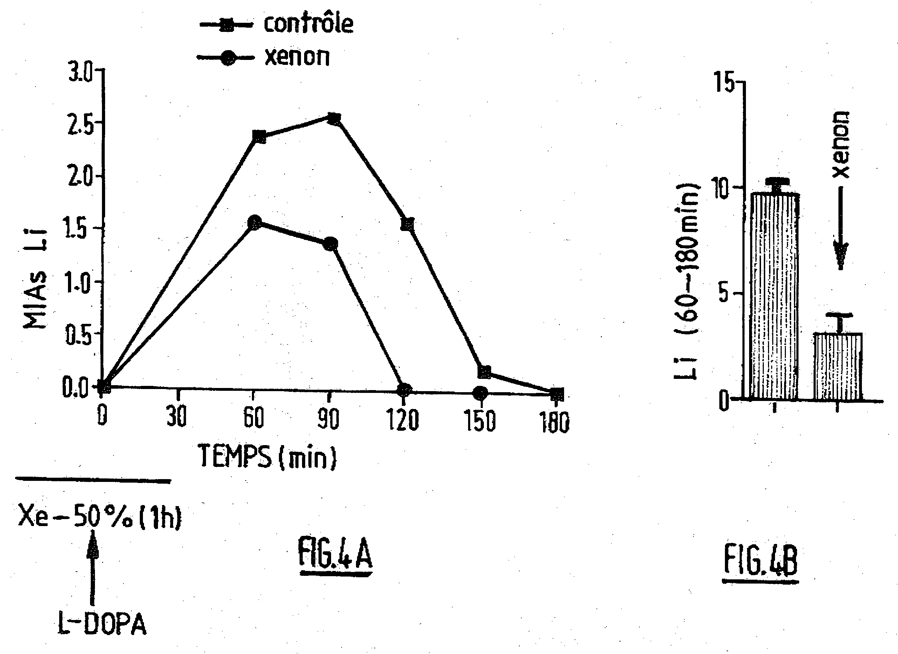

Les Figures 4A et 4B illustrent, quant à elles, les effets du xénon sur les dyskinésies des membres inférieurs (Li) chez le rat parkinsonien. Comme on le voit en Figure 4A, la cinétique de l'effet du xénon sur les dyskinésies des membres inférieurs suite à l'administration de L-Dopa (à t=0) montre une diminution significative de ces dyskinésies, là encore, tant en amplitude qu'en durée, par rapport au groupe contrôle ayant inhalé le mélange gazeux ne contenant pas de xénon.

En Figure 4B, la moyenne des dyskinésies des membres inférieurs (Li) de 60 à 180min fait apparaître que dans l'ensemble les rats ayant respirés le mélange contenant du xénon présente moins de dyskinésie des membres inférieurs.

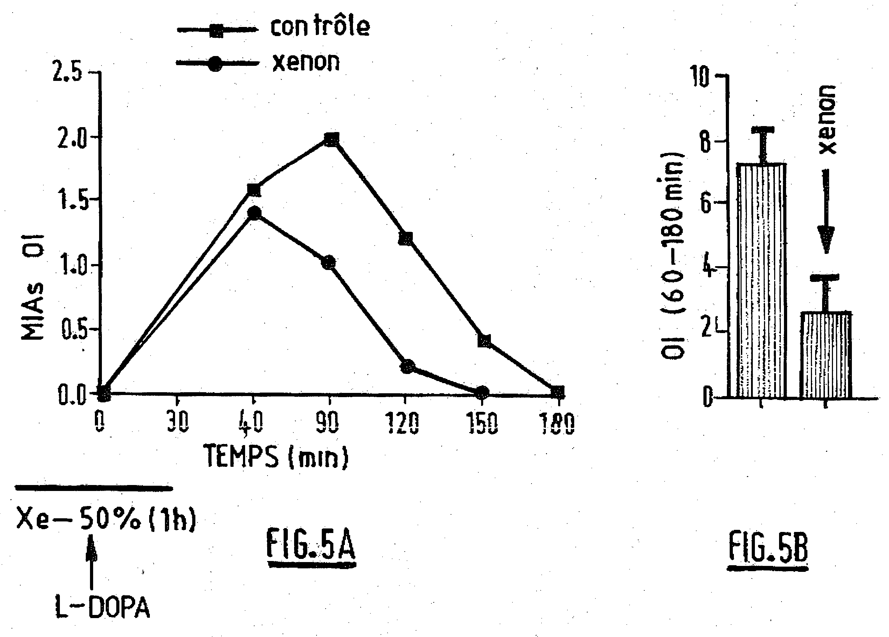

Les Figures 5A et 5B illustrent les effets du xénon sur les dyskinésies orolinguales (Ol) chez le rat parkinsonien. Comme on le voit en Figure 5A, la cinétique de l'effet du xénon sur les dyskinésies orolinguales (Ol) suite à l'administration de L-Dopa (à t=0) montre une diminution clair de ces dyskinésies, tant en amplitude qu'en durée, par rapport au groupé contrôle ayant respiré le mélange gazeux ne contenant pas de xénon.

En outre, en Figure 5B, la moyenne des dyskinésies orolinguales (Oi) de 60 à 180 min fait apparaître qu'en moyenne, les rats ayant inhalé le mélange contenant du xénon selon l'invention présente moins de dyskinésie orolinguales.

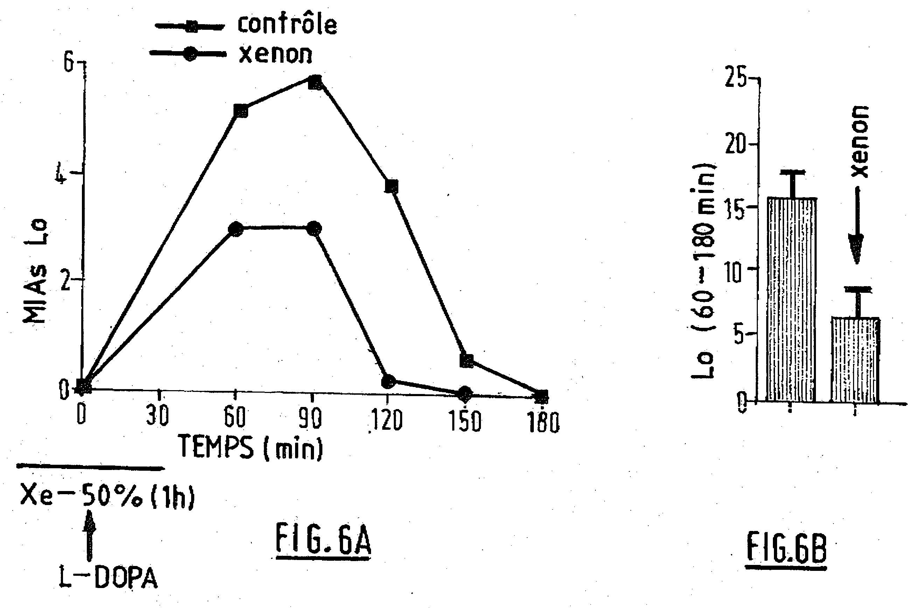

Enfin, les Figures 6A et 6B illustrent les effets du xénon sur les dyskinésies locomotrices (Lo) chez le rat parkinsonien. Comme on voit en Figure 6A, la cinétique de l'effet du xénon sur les dyskinésies locomotrices suite à l'administration de L-Dopa (à t=0) montre une diminution importante de ces dyskinésies, là aussi, tant en amplitude qu'en durée, par rapport au groupe contrôle ayant respiré le mélange gazeux ne contenant pas de xénon.

Là encore, la moyenne des dyskinésies locomotrices de 60 à 180 min fait apparaître, comme montré en Figure 6B, que globalement, les rats ayant respirés le mélange contenant du xénon présente moins de dyskinésie locomotrices.

Au final, chez le groupe d'animaux respirant un mélange gazeux du xénon/O₂ (50%/50% en volume), l'apparition des 4 types de dyskinésies est fortement réduite. Cette réduction est visible à la fois dans l'amplitude et dans la durée des dyskinésies.

Les résultats d'essais obtenus montrent que le xénon est un agent efficace contre les dyskinésies induites par la lévodopa et que ce gaz peut dès lors être utilisé dans le cadre d'une méthode de traitement thérapeutique des dyskinésies chez l'être humain.

Selon l'invention, on propose donc d'utiliser du xénon gazeux administré par inhalation pour traiter ou prévenir les dyskinésies chez le mammifère, en particulier chez l'être humain, et plus spécifiquement les dyskinésies induites par la lévodopa.

Toutefois, il est à souligner encore que le médicament inhalable à base de xénon selon l'invention n'est pas destiné à traiter à proprement parler la maladie de Parkinson en elle-même mais permet d'éviter, de minimiser ou de traiter l'apparition d'une dyskinésie pouvant résulter ou susceptible d'être induite par un composé servant au traitement de la maladie de Parkinson, en particulier la lévodopa.

D'une façon générale, lors d'un traitement dans le cadre de la présente invention utilisant un médicament gazeux à base de xénon gazeux, l'administration de xénon chez l'homme peut se faire au moyen d'un ventilateur, d'un nébuliseur ou spontanément avec des bouteilles préconditionnées, raccordés à un masque facial ou nasal, ou des lunettes nasales.

La durée d'administration sera choisie au cas par cas en fonction de l'importance de la dyskinésie affectant le patient considéré, par exemple le xénon pourra être administré pendant une durée de quelques minutes à quelques dizaines de minutes, voire d'heures, par exemple moins d'une heure, et à une fréquence pouvant atteindre une à plusieurs fois par jour ou par semaine, par exemple 1 fois par jour pendant 2 semaines.

L'efficacité du traitement pourra être évaluée en comptabilisant le nombre et/ou la fréquence par exemple des dyskinésies induites par la lévodopa, survenant chez l'homme pendant une période donnée et en comparant ce nombre avec des valeurs de références.

Le xénon ou mélange gazeux à base de xénon est préférentiellement conditionné en bouteille de gaz sous pression ou sous forme liquide, par exemple dans une bouteille d'un à plusieurs litres (contenant en eau) et à une pression comprise entre 2 et 300 bar.

Le xénon ou mélange gazeux à base de xénon peut se présenter sous forme « prêt à l'emploi », par exemple en pré-mélange avec de l'oxygène, ou alors être mélangé sur site lors de son utilisation, notamment avec de l'oxygène et éventuellement un autre composé gazeux ou en combinaison avec la lévodopa, comme expliqué ci-avant.

## Revendications

1. Médicament gazeux à base de xénon gazeux pour une utilisation par inhalation pour traiter ou prévenir une dyskinésie chez le mammifère, ladite dyskinésie étant induite par la lévodopa (L-DOPA).

2. Médicament pour une utilisation selon la revendication précédente, **caractérisé en ce que** le mammifère est un être humain.

3. Médicament pour une utilisation selon l'une des revendications précédentes, **caractérisé en ce que** la dyskinésie est induite par le traitement, utilisant la lévodopa, de la maladie de Parkinson.

4. Médicament pour une utilisation selon l'une des revendications précédentes, **caractérisé en ce que** le xénon gazeux est mélangé avec un gaz contenant de l'oxygène.

5. Médicament pour une utilisation selon l'une des revendications précédentes, **caractérisé en ce qu'**il contient une proportion volumique de xénon comprise entre 5 et 50%.

6. Médicament pour une utilisation selon la revendication 5, **caractérisé en ce qu'**il contient une proportion volumique de xénon comprise entre 5 et 40%.

7. Médicament pour une utilisation selon l'une des revendications précédentes, **caractérisé en ce que** le xénon est mélangé avec au moins 21% en volume d'oxygène.

8. Médicament pour une utilisation selon l'une des revendications précédentes, **caractérisé en ce que** le xénon est mélangé avec au moins un autre composé choisi parmi le protoxyde d'azote, l'argon, hélium, néon, krypton, H₂S, CO, NO et l'azote.

9. Médicament pour une utilisation selon l'une des revendications précédentes, **caractérisé en ce que** le xénon est administré en combinaison avec la lévodopa.

10. Médicament pour une utilisation selon l'une des revendications précédentes, **caractérisé en ce que** le xénon est administré préalablement, simultanément et/ou postérieurement à l'administration de la lévodopa.

11. Médicament pour une utilisation selon la revendication 4, **caractérisé en ce que** le xénon gazeux est mélangé avec de l'air ou un mélange N₂/O₂.

12. Médicament pour une utilisation selon la revendication 6, **caractérisé en ce qu'**il contient une proportion volumique de xénon comprise entre 10 et 30%.

## Patentansprüche

1. Gasförmiges Arzneimittel auf der Basis von gasförmigem Xenon für eine Verwendung zur Behandlung oder Vorbeugung einer Dyskinesie beim Säugetier, wobei die Dyskinesie durch Levodopa (L-DOPA) bewirkt wird.

2. Arzneimittel für eine Verwendung nach dem vorhergehenden Anspruch, **dadurch gekennzeichnet, dass** das Säugetier ein Mensch ist.

3. Arzneimittel für eine Verwendung nach einem der vorhergehenden Ansprüche, **dadurch gekennzeichnet, dass** die Dyskinesie durch die Behandlung der Parkinson-Krankheit mit Levodopa bewirkt wird.

4. Arzneimittel für eine Verwendung nach einem der vorhergehenden Ansprüche, **dadurch gekennzeichnet, dass** das gasförmige Xenon mit einem Gas gemischt ist, das Sauerstoff enthält.

5. Arzneimittel für eine Verwendung nach einem der vorhergehenden Ansprüche, **dadurch gekennzeichnet, dass** es einen Volumenanteil an Xenon zwischen 5 und 50 % enthält.

6. Arzneimittel für eine Verwendung nach Anspruch 5, **dadurch gekennzeichnet, dass** es einen Volumenanteil an Xenon zwischen 5 und 40 % enthält.

7. Arzneimittel für eine Verwendung nach einem der vorhergehenden Ansprüche, **dadurch gekennzeichnet, dass** das Xenon mit wenigstens 21 Volumen-% Sauerstoff gemischt ist.

8. Arzneimittel für eine Verwendung nach einem der vorhergehenden Ansprüche, **dadurch gekennzeichnet, dass** das Xenon mit wenigstens einer weiteren Verbindung gemischt ist, die aus Distickstoffoxid, Argon, Helium, Neon, Krypton, H₂S, CO, NO und Stickstoff ausgewählt ist.

9. Arzneimittel für eine Verwendung nach einem der vorhergehenden Ansprüche, **dadurch gekennzeichnet, dass** das Xenon in Kombination mit Levodopa verabreicht wird.

10. Arzneimittel für eine Verwendung nach einem der vorhergehenden Ansprüche, **dadurch gekennzeichnet, dass** das Xenon vor, gleichzeitig mit und/oder nach der Verabreichung von Levodopa verabreicht wird.

11. Arzneimittel für eine Verwendung nach Anspruch 4, **dadurch gekennzeichnet, dass** das gasförmige Xenon mit Luft oder einem N₂/O₂-Gemisch gemischt ist.

12. Arzneimittel für eine Verwendung nach Anspruch 6, **dadurch gekennzeichnet, dass** es einen Volumenanteil an Xenon zwischen 10 und 30 % enthält.

## Claims

1. Gaseous drug based on gaseous xenon for use by inhalation to treat or prevent dyskinesia in mammals, said dyskinesia being induced by levodopa (L-DOPA).

2. Drug for use according to the preceding claim, **characterised in that** the mammal is a human being.

3. Drug for use according to either of the preceding claims, **characterised in that** the dyskinesia results from the treatment, which uses levodopa, of Parkinson's disease.

4. Drug for use according to any of the preceding claims, **characterised in that** the gaseous xenon is mixed with a gas containing oxygen.

5. Drug for use according to any of the preceding claims, **characterised in that** the drug contains a proportion by volume of xenon of between 5 and 50 %.

6. Drug for use according to claim 5, **characterised in that** the drug contains a proportion by volume of xenon of between 5 and 40 %.

7. Drug for use according to any of the preceding claims, **characterised in that** the xenon is mixed with at least 21 vol.% oxygen.

8. Drug for use according to any of the preceding claims, **characterised in that** the xenon is mixed with at least one other compound selected from nitrous oxide, argon, helium, neon, krypton, H₂S, CO, NO and nitrogen.

9. Drug for use according to any of the preceding claims, **characterised in that** the xenon is administered in combination with levodopa.

10. Drug for use according to any of the preceding claims, **characterised in that** the xenon is administered prior to, at the same time as and/or subsequently to administration of levodopa.

11. Drug for use according to claim 4, **characterised in that** the gaseous xenon is mixed with air or a N₂/O₂ mixture.

12. Drug for use according to claim 6, **characterised in that** the drug contains a proportion by volume of xenon of between 10 and 30 %.
